# EUROPEAN PATENT APPLICATION

(11) **EP 4 579 077 A1**
(43) Date of publication of application: **02.07.2025**
(21) Application number: 24223363.3
(22) Date of filing: 27.12.2024
(51) Int. Cl.: F04B 9/12, A61M 5/145

(54) **FLUID PROCESSING SYSTEM WITH HIGH PRESSURE PNEUMATIC SYRINGE PUMP**

(30) Priority: 27.12.2023 US 202363615004 P
(71) Applicant: Fenwal, Inc., Lake Zurich, IL 60047 (US)
(72) Inventor: MADSEN, James Gregory, Lake Zurich, 60047 (US); WEGENER, Christopher J., Lake Zurich, 60047 (US); DODGE, Alexander, Lake Zurich, 60047 (US)
(74) Representative: Maikowski & Ninnemann Patentanwälte Partnerschaft mbB

(57) **Abstract**

A fluid processing system includes a syringe having a barrel and a plunger configured for movement within the barrel, a housing, a syringe support arranged on the housing configured to support the syringe, and a syringe clamp connected to the housing and configured to clamp the syringe to the syringe support. The syringe clamp is connected to the housing and includes a sealing section for sealing against the syringe and a holding section for latching the syringe clamp in a clamped condition. The sealing section is configured to apply a clamping force to a top face of the syringe to form a seal. The holding section includes a clamping surface configured to engage a clamp bearing surface of the syringe support to secure the syringe clamp in the clamped condition.

## Description

### Background

### Field of the Disclosure

The disclosure relates to a fluid processing system. More particularly, the disclosure relates to a high-pressure pneumatic syringe pump system for driving microfluidic chips.

### Description of Related Art

Microfluidic chips offer novel ways to use micron-sized features within a fluid path to achieve physical fluid flow conditions that are not possible using macro-sized features. One relevant use of microfluidic chips is for separation of blood or blood components. This can be achieved through varied approaches (e.g., using inertial techniques or electric or gravitational separation field), which often enable much more precise separation than can be achieved through traditional means, such as macro-scale centrifugation or filtration.

A known microfluidic chip may include a fluid compartment configured as a microfluidic channel having a width or thickness on the order of approximately 50-500 µm. Fluid, e.g., blood cell suspensions, may be provided to the microfluidic channel and a pump may be operated to drive the blood cell suspension through the microfluidic channel. Due to the width or thickness of the microfluidic channel relative to the size of cellular components (for example) of the blood cell suspension, the cellular components may be separated from the blood cell suspension as the pump drives the fluid. In such a process, non-cellular components (e.g., plasma) or cellular components of a relatively smaller size, from which cellular components of a relatively larger size have been separated, may continue to flow through microfluidic channel for collection.

Currently, peristaltic pumps or traditional syringe pumps are used for driving microfluidic chips, i.e., for pumping the fluid to be treated through the microfluidic chip. Such pumps are suitable for relatively low-pressure applications. However, driving fluid through microfluidic chips often requires relatively higher pressure, for example, up to about 50 psi, and in some applications, up to about 100 psi.

To achieve such relatively high pressures, e.g., up to about 50 psi or 100 psi, the peristaltic pumps and traditional syringe pumps must be used together with a pressure chamber. The pressure chamber may be a clamshell design, made of aluminum, for example, and pressurized by an external source. However, when using a pressure chamber in microfluidics, a volume of source material (e.g., a fluid to be treated) is limited by the initial source container (e.g., a fluid bag) and a size of the chamber. For example, a pressure chamber volume required for treating 200 ml of source material is twice as large as the pressure chamber volume for treating 100 ml of source material.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a fluid processing system includes at least one syringe having a barrel and a plunger configured for reciprocal movement within the barrel, wherein the syringe includes a top face and a top flange at a proximal end and a tip at a distal end, a pump housing, at least one syringe support arranged on the pump housing having a support surface configured engage the top flange to support the syringe in a fluid processing procedure, and at least one syringe clamp connected to the housing, the syringe clamp comprising a sealing section connected to the housing with a first hinge and a holding section connected to the sealing section with a second hinge, the syringe clamp moveable between an unclamped condition and a clamped condition. The sealing section includes a sealing element on a sealing surface and is pivotable on the first hinge between a raised condition and a lowered condition in which the sealing section is configured to apply a clamping force to the top face of the syringe to form a seal against the top face with the sealing element. The holding section is pivotable on the second hinge between an unlatched condition and a latched condition engaged with the syringe support. Movement of the holding section to the latched condition causes movement of the syringe clamp to the clamped condition by applying a closing force to the sealing section through the second hinge such that the sealing section applies the clamping force to the top face, and movement of the holding section to the unlatched position causes movement of the syringe clamp to the unclamped condition by releasing the closing force and the clamping force.

In another aspect, a syringe pump assembly includes a pump housing, at least one syringe support arranged on the pump housing having a support surface configured to support a syringe in a fluid processing procedure, and a corresponding at least one syringe clamp connected to the housing, the syringe clamp comprising a sealing section connected to the housing with a first hinge and a holding section connected to the sealing section with a second hinge, the syringe clamp moveable between an unclamped condition and a clamped condition. The sealing section includes a sealing element on a sealing surface and is pivotable on the first hinge between a raised condition corresponding to the unclamped condition and a lowered condition. The holding section is pivotable on the second hinge between an unlatched condition corresponding to the unclamped condition and a latched condition engaged with the syringe support and corresponding to the clamped condition. Movement of the holding section to the latched condition causes movement of the syringe clamp to the clamped condition by applying a closing force to the sealing section through the second hinge, and movement of the holding section to the unlatched condition causes movement of the syringe clamp to the unclamped condition by releasing the closing force.

### Brief Description of the Drawings

Fig. 1 is a perspective view of an exemplary syringe pump assembly and fluid processing system, in accordance with an aspect of the present disclosure;
Fig. 2 is a perspective view illustrating an example of a syringe pump assembly having a syringe clamp in an unclamped condition;
Fig. 3 is a perspective view illustrating a fluid processing system having the syringe pump assembly of Fig. 2, including a syringe arranged on the syringe support;
Fig. 4 is a perspective view illustrating the fluid processing system similar to the view of Fig. 3;
Fig. 5 is a perspective view illustrating the syringe pump assembly of Figs. 3 and 4 with the syringe clamp in the clamped condition to hold a syringe on the support;
Fig. 6 is a side view of the syringe pump assembly of Fig. 2 having a syringe clamp with a holding section in an unlatched condition;
Fig. 7 is a side view of the syringe pump assembly of Fig. 6, with the holding section in a latched condition;
Fig. 8 is a side view of the syringe pump assembly of Fig. 2 having the syringe clamp in the unclamped condition;
Fig. 9 is a side view of the syringe pump assembly of Fig. 8, having the syringe clamp in a clamped condition;
Fig. 10 is a cross-sectional side view of the syringe pump assembly of Fig. 2, having the syringe clamp in the unclamped condition;
Fig. 11 is a cross-sectional side view of the syringe pump assembly of Fig. 10, having the syringe clamp in the clamped condition;
Fig. 12 is a front view of the syringe pump assembly of Fig. 2, having the syringe clamp in a clamped condition; and
Fig. 13 is a front cross-sectional view of the syringe pump assembly of Fig. 12, having the syringe clamp in the clamped condition..

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

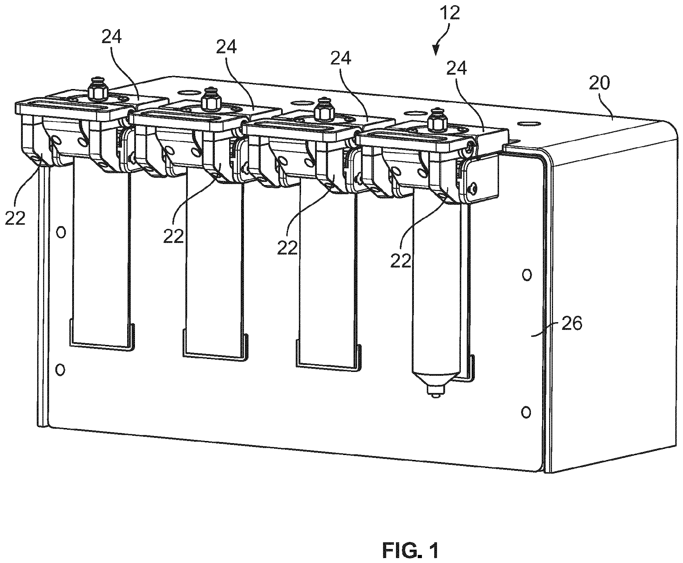

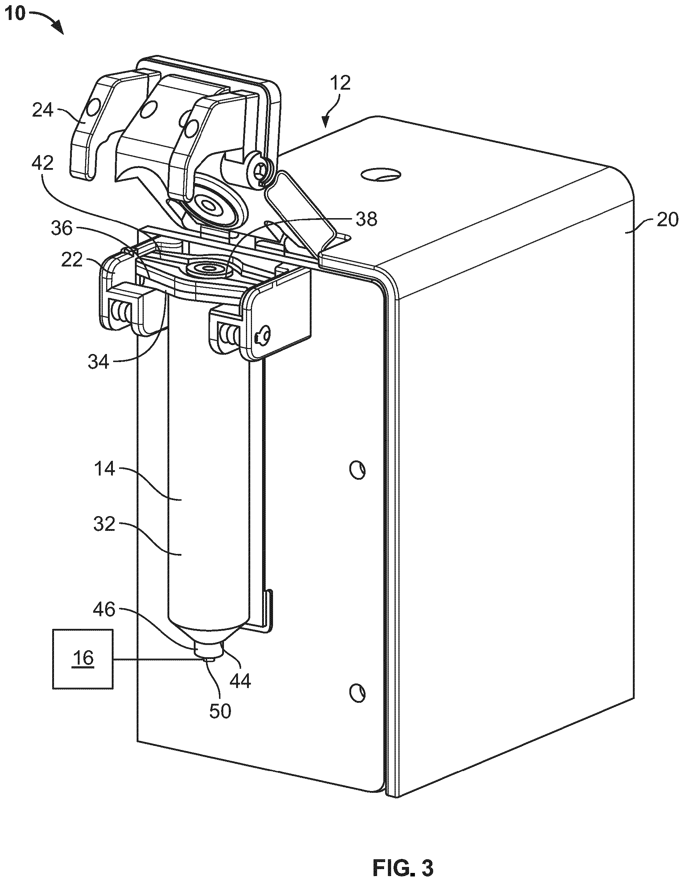

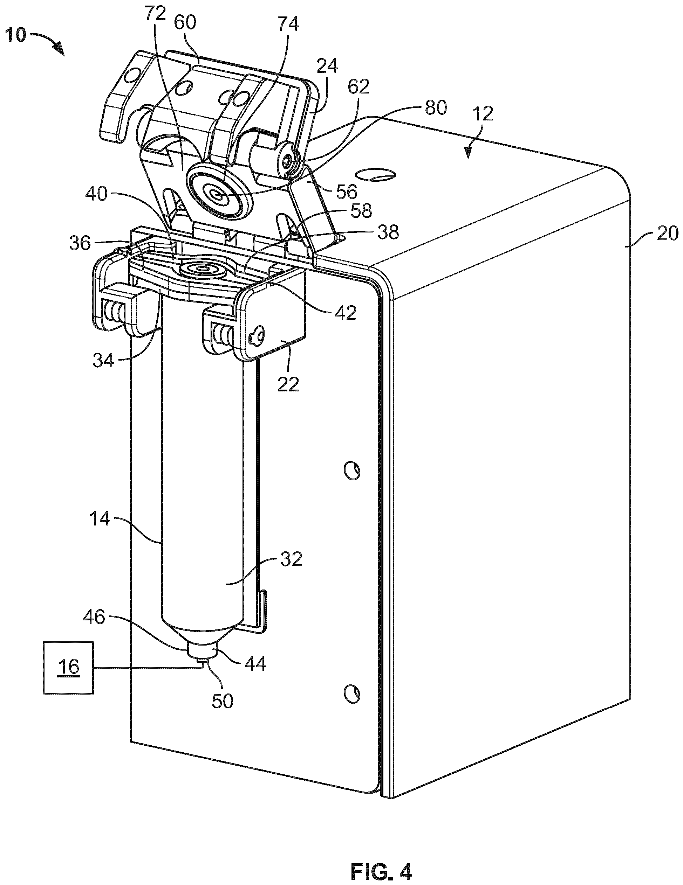

Referring generally to Figs. 1-4 a fluid processing system 10 having at least one syringe pump assembly 12 configured to drive a fluidically connected microfluidic chip 16 (schematically shown in Figs. 3 and 4) at pressures up to about 50 psi, and in some examples, up to about 100 psi. In the illustrated example, the microfluidic chip 16 is schematically shown directly connected to a tip of a syringe in a syringe pump assembly 12. However, it will be appreciated that the present examples are not limited to such a configuration and that the microfluidic chip 16 may be indirectly connected to the syringe in a suitable manner, while being configured to receive fluid from the syringe. As described in the various examples, the fluid processing system 10 includes housing (also referred to as a pump housing) 20, at least one syringe support 22 arranged on the housing 20 for supporting a syringe, and a corresponding at least one syringe clamp 24 movable between an unclamped condition in which the syringe may be freely removed from or placed on the syringe support 22, and a clamped condition in which the syringe is secured, or clamped between the syringe support 22 and the syringe clamp 24.

Referring now to Fig. 1, in one example, the fluid processing system 10 generally includes the syringe pump assembly 12, a syringe 14 arranged on the syringe pump assembly 12, and optionally, the microfluidic chip 16 (see Figs. 3 and 4) fluidically connected to the syringe 14. The syringe pump assembly 12 according to various examples herein includes the housing 20 (also referred to as a "pump housing"), the syringe support 22 arranged on the housing 20 and the syringe clamp 24 rotatably connected to the housing 20. As described further below, the syringe pump assembly 12 is configured to clamp a portion of the syringe 14 between the syringe support 22 and the syringe clamp 24 while holding the syringe 14 against unintended movements and withstanding pressures experienced during a fluid treatment process .

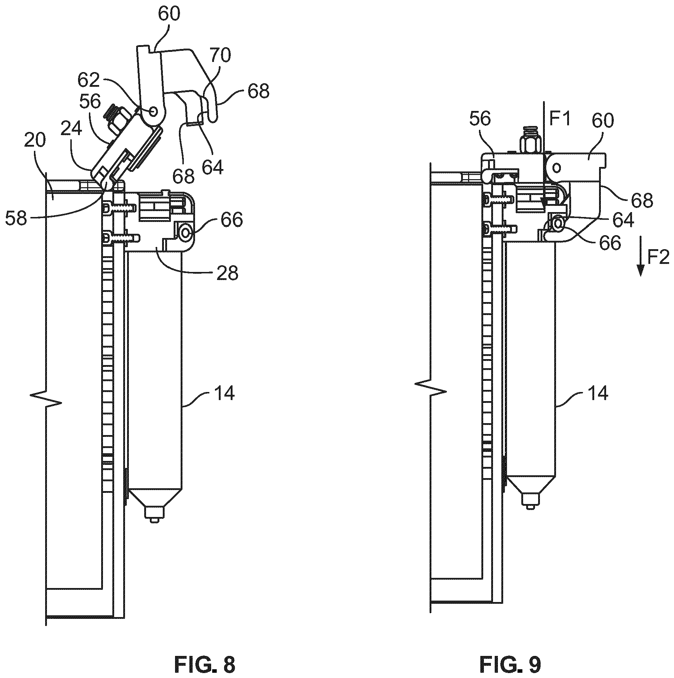

The housing 20 may include one or more syringe support 22. The one or more syringe support 22 may be arranged on a plate 26 of the housing 20 and may be secured to the housing 20 by one or more fasteners as shown in Figs. 8 and 9. In the illustrated example, four syringe supports 22 are arranged on the plate 26 with corresponding syringe clamps 24. However, it will be appreciated that the number of syringe supports 22 and clamps 24 arranged on the housing 20 is not limited to this example.

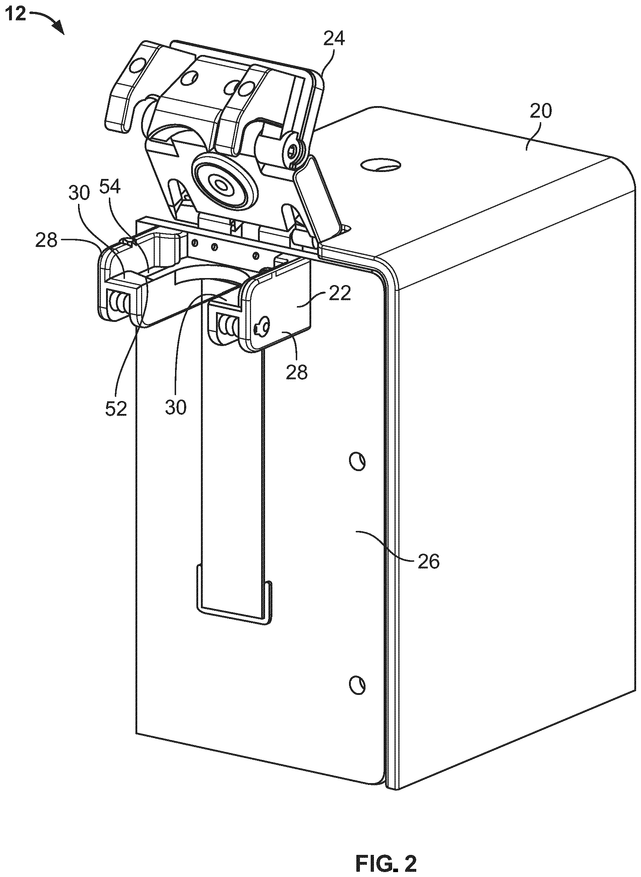

Referring now to Figs. 1 and 2, the syringe clamp 24 is movable between a closed, or clamped condition (see Fig. 1), and an open, or unclamped condition (Fig. 2). In the open or unclamped condition, the syringe clamp 24 is spaced from the syringe support 22 such that the syringe 14 (see Fig. 1) can be freely removed from, or placed onto, the syringe support 22. The syringe support 22 may include spaced apart support arms 28 between which the syringe 14 may be received. Each support arm 28 may include a support surface 30 for supporting at least a portion of the syringe 14.

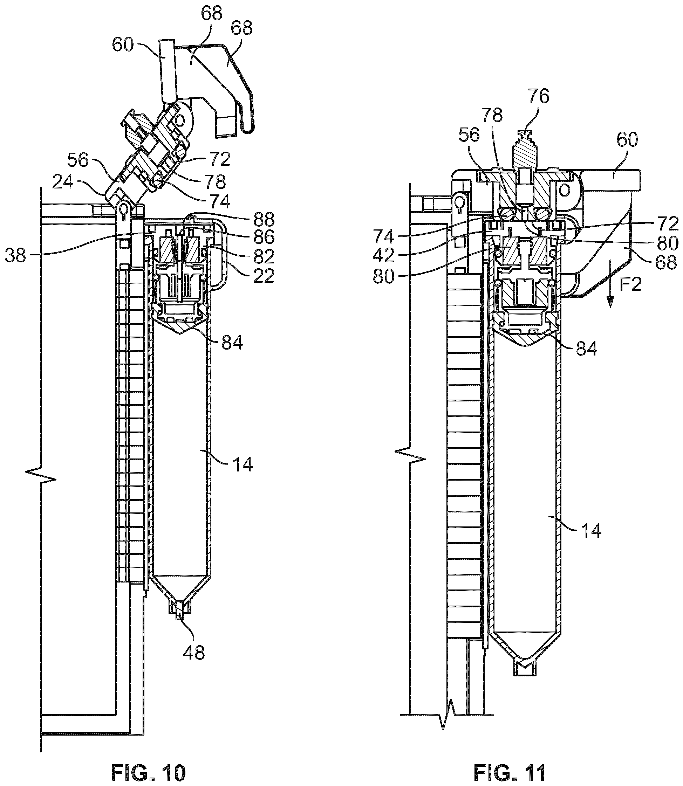

Referring now to Figs. 2-4, with the syringe clamp 24 open (i.e., in an unclamped condition), the syringe 14 may be arranged on or removed from the syringe support 22. In one example, the syringe 14 includes an elongated barrel 32, a barrel flange 34 at a proximal end 36, and a cap 38 connected to the barrel 32 at the proximal end 36. The barrel flange 34 extends laterally outward relative to the barrel 32. The cap 38 includes a top face 40 at an upper, most proximal end of the syringe 14, and a cap flange 42 extending laterally outward relative to the barrel 32. In the illustrated example, the barrel flange 34 and the cap flange 42 both extend laterally outward relative to the barrel 32 in at least two directions, e.g., from opposite sides of the barrel 32. In addition, the cap flange 42 may at least partially cover the barrel flange 34 at the proximal end 36. The syringe 14 also includes a tip 44 at a distal end 46 having a fluid passage 48 (see Fig. 10) to accommodate fluid flow into and out of the barrel 32. In some examples, the tip 44 may be provided with a luer connector 50 as well.

As shown in Figs. 3 and 4, the syringe 14 may be arranged on the syringe support 22 by situating the syringe 14 between the support arms 28 with the barrel flange 34 and cap flange 42 arranged on the support surfaces 30 of the support arms 28. In one example, as shown in Fig. 2, the support surfaces 30 are provided with a shoulder 52 which forms a recessed seat 54 in which the barrel flange 34 may be seated. In this manner, the syringe 14 may be properly positioned relative to the syringe support 22. In addition, the shoulder 52 may serve to restrain movement of the barrel flange 34, and in turn, the syringe 14 in one or more lateral directions.

With reference to Figs. 2-5, the syringe clamp 24 is rotatably connected to the housing 20. In one example, the syringe clamp 24 includes a first section (also referred to as a "sealing section" herein) 56 rotatably connected to the housing 20 by a first hinge 58. The syringe clamp 24 also includes a second section (also referred to as a "holding section" herein) 60 rotatably connected to first section 56 by a second hinge 62. As described further below, in the clamped condition, the first section 56 is configured to be sealed against the top face 40 to accommodate a fluidic connection between the syringe clamp 24 and the syringe 14. The second section 60, in the clamped condition, is configured to engage the syringe support 22 to hold the syringe clamp 24 in the clamped condition.

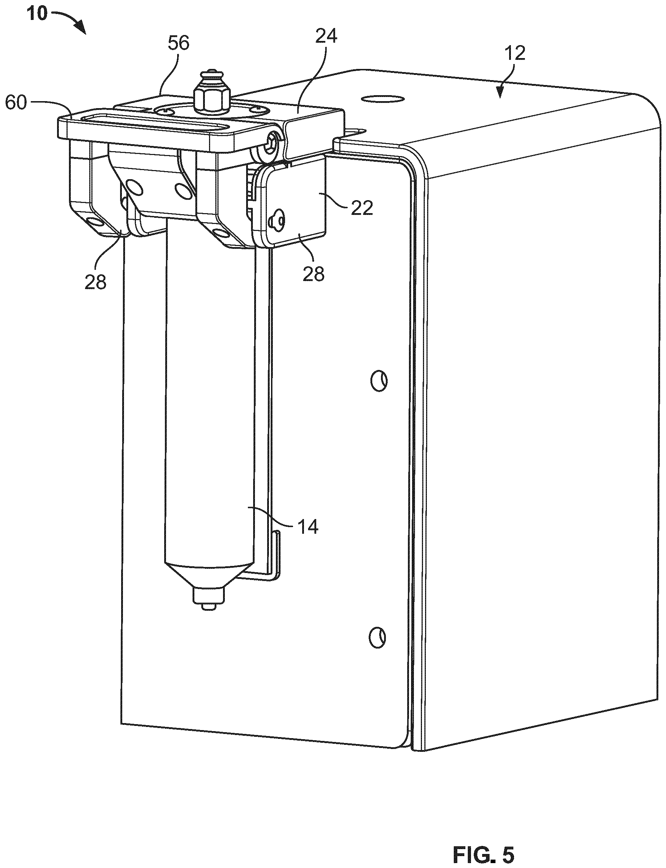

Referring now to Figs. 2-7, the first section 56 is moveable between a raised condition (Figs. 2-4) and a lowered condition (Figs. 5-7). In the raised condition, the first section 56 is spaced from the top face 40 of the cap 38 and the syringe support 22, which corresponds to the unclamped condition of the syringe clamp 24. In the lowered condition, the first section 56 is rotated from the raised condition toward the syringe supports 22 and into contact with the top face 40 of the cap 38 (when the syringe 14 is arranged on the syringe support 22). The first section 56 may be in the lowered condition in both the unclamped and clamped conditions of the syringe clamp 24, depending on the position of the second section 60, described below.

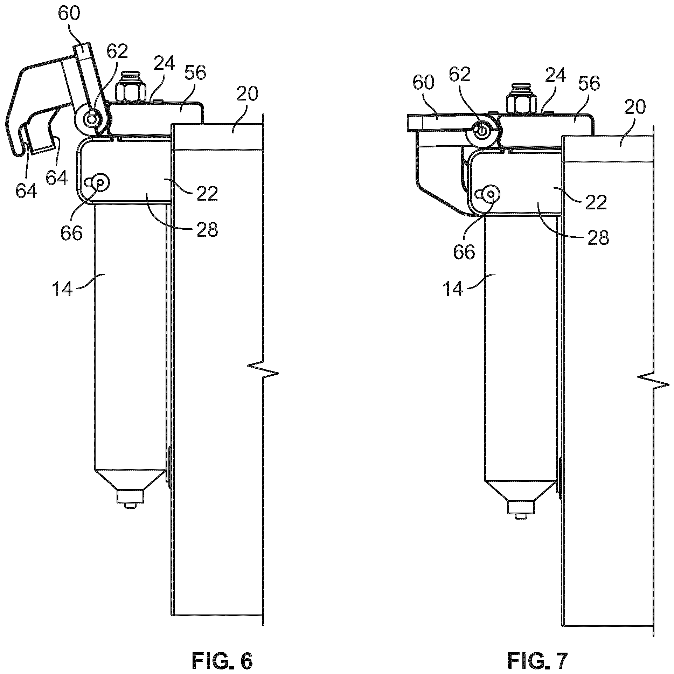

With further reference to Figs. 2-7, the second section 60 is movable between an unlatched condition (Figs. 2-4 and 6) and a latched condition (Figs. 5 and 7). In the unlatched condition, the second section 60 is rotatably disengaged or spaced from the syringe support 22, which corresponds to the unclamped condition of the syringe clamp 24. The second section 60 may be in the unlatched condition with the first section 56 in either of the raised condition (Figs. 2-4) or the lowered condition (Fig. 6). In the latched condition, the second section 60 is rotated into engagement with the syringe support 22. The second section 60 includes at least one clamping surface 64 configured to engage a corresponding clamp bearing surface 66 of the syringe support 22.

In the illustrated example, the second section 60 includes two clamping arms 68 and a clamping surface 64 is provided on each clamping arm 68. Also in the illustrated example, the syringe support 22 includes two clamp bearing surfaces 66, for example, arranged on respective support arms 28. Accordingly, the clamping surfaces 64 on clamping arms 68 are configured to engage the corresponding clamp bearing surfaces 66 on support arms 28.

The clamping surface 64 may be arranged on an upper portion of a clamping arm 68 or respective clamping arms 68 of the second section 60. The clamp bearing surface 66 may be arranged on an underside of the syringe support 22 or respective support arms 28. Thus, in the latched condition, the clamping surface or surfaces 64 are engaged with a corresponding clamp bearing surface or surfaces 66 on the underside of the syringe support 22.

Referring to Figs. 6 and 7, with the first section 56 in the lowered condition and the second section 60 in the unlatched condition (Fig. 6), the syringe clamp 24 is in the unclamped condition and the first section 56 is not sealed against the top face 40 of the syringe cap 38 (see Fig. 4). With the first section 56 in the lowered condition and the second section 60 in the latched condition (Fig. 7), the syringe clamp 24 is in the clamped condition and the first section 56 is sealed against the top face 40 of the syringe cap 38 to facilitate air flow from a supply to the syringe 14 via the first section 56, as described further below.

As shown in Figs. 8 and 9, the clamp bearing surface 66 of the syringe support 22 may be implemented as bearing element, such as a roller or ball bearing. In some examples, the clamping surface 64 of the second section 60 includes a detent 70 configured to receive the bearing element 66. Thus, with the second section 60 in the latched condition (and with the syringe clamp 24 in the clamped condition), the bearing element 66 is positioned in the detent 70 to hold the second section 60 in the latched condition and the syringe clamp 24 in the clamped condition. By way of engagement and disengagement of the bearing element 66 and detent 70, a user may perceive an audible and/or a tactile "click" when latching or unlatching the second section 60.

Further, in the clamped condition, the second section 60 may resist movement out of the latched condition by way of interaction between the clamping surface 64 and the clamp bearing surface 66. For example, a holding force to resist movement out of the latched condition may be provided by the bearing element 66 positioned in the detent 70.

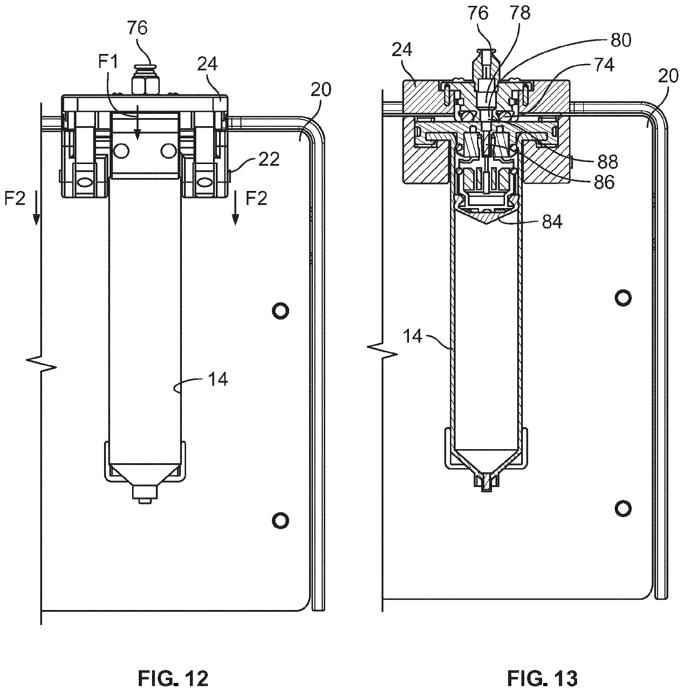

Referring now to Figs. 3, 4 and 10-13, the first section 56 includes a sealing surface 72 on its lower surface, to be arranged in opposition to the top face 40. A sealing element 74 is arranged on the sealing surface 72 and is configured to form a seal between the first section 46 and the top face 40 when the syringe clamp 24 is in the clamped condition. In one example, the sealing element 74 is implemented as an O-ring.

As shown in Figs. 4, 10 and 11, for example, the first section 56 includes a pneumatic fitting 76 configured to be connected to an air supply and facilitate air flow in the first section 56. With reference to Figs. 10, 11 and 13, the first section 56 also includes a first channel 78 fluidically connected to the fitting 76 and a first channel opening 80 arranged on the sealing surface 72. The sealing element 74 substantially surrounds the first channel opening 80 on the sealing surface 72.

Still referring to Figs. 10, 11 and 13, the cap 38 may include a body portion 82 at least partially disposed within the barrel 32 at the proximal end 36. The cap flange 42 is arranged outside of the barrel 32 and extends from the body portion 82. The syringe 14 further includes a plunger 84 arranged in the barrel 32 and configured to move axially back and forth within the barrel 32 to draw fluid into the barrel 32 via fluid passage 48 or to expel fluid from the barrel 32 depending on the direction of movement.

The cap 38 includes a second channel 86 and a second channel opening 88 fluidically connected to the second channel 86. The second channel opening 88 is substantially surrounded by the sealing element 74 in the clamped configuration. Accordingly, with the syringe clamp 24 in the clamped configuration, the first channel 78 is fluidically connected to the second channel 84 via the first and second channel openings 80, 88 and a sealed connection formed between the sealing surface 72 and top face 40 by the sealing element 74.

In use, the first section 56 may act as an air supply block in the clamped configuration. For example, the pneumatic fitting 76 may be fluidically connected to an air supply (not shown) such that process air may flow in the first channel 78 and through the first channel opening 80 of the first section 56. Process air may flow between the second channel 84 and the first channel 78 via the first and second channel openings 80, 88 and the sealed connection formed by the sealing element 74.

The cap 38 is fluidically connected to the plunger 84 such that the process air interacts with the plunger 84 to drive the plunger 84 within the barrel 32. The process air may be introduced to the syringe at either a positive or negative pressure. Positive air pressure drives the plunger 84 toward the tip 44 to expel fluid from the barrel 32, with negative air pressure retracts the plunger 84 and draws fluid into the barrel 32. Positive air pressure may be used to drive fluid through a microfluidic chip, while negative air pressure may be used to draw fluid out of the microfluidic chip, for example, to prime the microfluidic chip. In one example, the negative pressure allows for priming the microfluidic chip by removing air before processing a source material or fluid, such as cellular material. The negative pressure may also enable the syringe 14 to draw fluid from a source container, e.g., a bag, to then process through the microfluidic system.

With reference to Figs, 9, 11 and 12, in the clamped condition, the first section 56 applies a clamping force F1 against the top face 40 to hold the sealing element 74 against the top face 40 and thereby form the sealed connection for air flow between the air supply and the syringe 14. The clamping force F1 is applied by the first section 56 in response to a closing force F2 applied to the first section 56 by the second section 60. The closing force F2 is provided in response to interaction between the second section 60 and the syringe support 22 when moving the second section 60 into the latched condition (i.e., when moving the syringe clamp 24 from the unclamped condition to the clamped condition). That is, the closing force F2 may be provided by a reaction force from the syringe support 22 to the second section 60, which in turn, is applied to the first section 56. As shown in the figures, the closing force F2 is a substantially downward force applied through the second section 60 to the first section 56, which causes a moment on the first section 56. The closing force F2 (and associated moment) urges the first section 56 toward the top face 40 such that the first section 56 applies the clamping force F1 to the top face 40 via the sealing element 74.

The closing force F2 and clamping force F1 are released by moving the second section 60 from the latched condition to the unlatched condition, thereby moving the syringe clamp 24 to the unclamped condition.

In the illustrated examples, the second section 60 is configured to resist movement out of the latched condition in response to various pressures applied to or within the syringe 14 during pneumatic operation. For example, the plunger 84 may be operated to move within the barrel 32 by the process air introduced to the syringe 14 as described above. By varying the air pressure of the process air, the syringe 14 can be pressurized within the range of about -10 psi up to about 50 psi, and other examples, up to about 100 psi. In this manner, the syringe 14, by way of the plunger 84, may be operated to provide fluid to the microfluidic chip 16 (shown schematically in Figs. 3 and 4) at a sufficiently high pressure to drive the microfluidic chip 16.

Accordingly, in the various examples above, the syringe pump assembly 12 is configured to configured to operate a syringe 14 to drive fluid in the microfluidic chip 16 at relatively high pressures, such as up to about 50 psi in some applications, and up to about 100 psi in other applications. The syringe 14 may be pressurized via the seal formed at the top face 40 by the o-ring 74 which is connected to a pneumatic control. The o-ring 74 is pressed against the syringe 14 using an over-center hinge design. In the illustrated example, the syringe 14 may be operated under such relatively high pressures due, at least in part to the syringe clamp 24 clamping the syringe 14 on the syringe support 22 and in turn, securing the syringe against inadvertent movements under such pressures. To this end, the syringe clamp 24 is configured to resist movement out of the clamped configuration for example, by interaction between clamping surface 64 and the clamp bearing surface 66. Accordingly, the fluid processing system 10 and the syringe pump assembly 12 of the present examples may operate the syringe 14 to provide such relatively high pressures for driving a microfluidic chip 16 without using a pressure chamber of the type required to provide adequate pressure in conventional systems for driving microfluidic chips.

In the present examples, the high-pressure syringe pump assembly 12 and fluid processing system 10 allow for high pressure microfluidic processing in a form factor which permits the syringe 14 to be repeatably loaded and unloaded with source material (e.g., a fluid to be processed). This may allow for differing volume to be processed without be restrained by the size / volume of a pressure chamber. The syringe pump assembly of the present examples is applicable wherever volume-controlled processing is performed at high pressures. The present syringe pump can accurately dispense volumes at positive and negative pressures.

According to a non-limiting example, microfluidic processing described herein may refer to processing of a blood cell suspension. The blood cell suspension may be processed to separate cellular components based on size (i.e., < 5 um vs. > 5 um), such that cellular components having a relatively larger size may be separated from the blood cell suspension while cellular components having a relatively smaller size may be driven through the microfluidic chip 16. In other examples, all or substantially all of the cellular components may be separated from the blood cell suspension while plasma may be driven through the microfluidic chip 16.

### Aspects

Aspect 1. A fluid processing system comprising: at least one syringe having a barrel and a plunger configured for reciprocal movement within the barrel, wherein the syringe includes a top face and a top flange at a proximal end and a tip at a distal end; a pump housing; at least one syringe support arranged on the pump housing having a support surface configured engage the top flange to support the syringe in a fluid processing procedure; and at least one syringe clamp connected to the housing, the syringe clamp comprising a sealing section connected to the housing with a first hinge and a holding section connected to the sealing section with a second hinge, the syringe clamp moveable between an unclamped condition and a clamped condition, wherein the sealing section includes a sealing element on a sealing surface and is pivotable on the first hinge between a raised condition and a lowered condition in which the sealing section is configured to apply a clamping force to the top face of the syringe to form a seal against the top face with the sealing element, wherein the holding section is pivotable on the second hinge between an unlatched condition and a latched condition engaged with the syringe support, and wherein movement of the holding section to the latched condition causes movement of the syringe clamp to the clamped condition by applying a closing force to the sealing section through the second hinge such that the sealing section applies the clamping force to the top face, and movement of the holding section to the unlatched position causes movement of the syringe clamp to the unclamped condition by releasing the closing force and the clamping force.

Aspect 2. The fluid processing system according to Aspect 1, wherein the holding section includes a clamping surface and the syringe support includes a clamp bearing surface, wherein the clamping surface is configured to engage the clamp bearing surface such that movement of the holding section to the latched condition increases the clamping force applied by the sealing section.

Aspect 3. The fluid processing system according to Aspect 2, wherein the holding section further includes a clamping arm and the clamping surface is formed on the clamping arm and is configured to engage the clamp bearing surface at an underside of the syringe support in the latched condition.

Aspect 4. The fluid processing system according to Aspect 2, wherein: the syringe support further includes spaced apart support flanges configured to receive the barrel therebetween, wherein the clamp bearing surface includes clamp bearing surfaces formed on the respective support flanges, and the holding section further includes spaced apart clamping arms and the clamping surface includes clamping surfaces formed on the respective clamping arms, and the clamping surfaces are configured to engage corresponding clamp bearing surfaces at an underside of the support flanges in the latched condition.

Aspect 5. The fluid processing system according to any of Aspects 1-4, wherein the clamping surface includes a detent and the clamp bearing surface includes a bearing element, wherein the bearing element is positioned in the detent when the holding section is in the latched condition.

Aspect 6. The fluid processing system according to any of Aspects 1-5, wherein the sealing element is a sealing o-ring, and wherein the sealing o-ring is configured to be pressed against the top face under the clamping force in the clamped condition to form the seal.

Aspect 7. The fluid processing system according to any of Aspects 1-5, wherein the sealing section includes a pneumatic fitting, a first channel fluidically connected to the pneumatic fitting and a first channel opening arranged on the sealing surface and surrounded by the sealing element.

Aspect 8. The fluid processing system according to Aspect 7, wherein the top face includes a second channel having a second channel opening, and wherein the first channel and the second channel are fluidically connected in the clamped condition.

Aspect 9. The fluid processing system according to Aspect 8, wherein air is provided to the syringe via the first channel and the second channel to pressurize the syringe between about -10 PSI and about 100 PSI.

Aspect 10. The fluid processing system according to Aspect 9, comprising a plurality of syringes arranged on the pump housing.

Aspect 11. The fluid processing system according to any of Aspects 1-9, wherein the tip includes a tip opening for fluid flow into or out of the barrel in response to pressurization of the syringe.

Aspect 12. The fluid processing system according to any of Aspects 1-9, wherein the syringe is a disposable syringe.

Aspect 13. The fluid processing system according to any of Aspects 1-9, further comprising a microfluidic chip fluidically connected to the syringe.

Aspect 14. The fluid processing system according to Aspect 13, wherein the syringe is configured to be positively pressurized to drive fluid through the microfluidic chip.

Aspect 15. The fluid processing system according to Aspect 13, wherein the syringe is configured to be negatively pressurized for priming the microfluidic chip.

Aspect 16. A syringe pump assembly comprising: a pump housing; at least one syringe support arranged on the pump housing having a support surface configured to support a syringe in a fluid processing procedure; and a corresponding at least one syringe clamp connected to the housing, the syringe clamp comprising a sealing section connected to the housing with a first hinge and a holding section connected to the sealing section with a second hinge, the syringe clamp moveable between an unclamped condition and a clamped condition, wherein the sealing section includes a sealing element on a sealing surface and is pivotable on the first hinge between a raised condition corresponding to the unclamped condition and a lowered condition, wherein the holding section is pivotable on the second hinge between an unlatched condition corresponding to the unclamped condition and a latched condition engaged with the syringe support and corresponding to the clamped condition, and wherein movement of the holding section to the latched condition causes movement of the syringe clamp to the clamped condition by applying a closing force to the sealing section through the second hinge, and movement of the holding section to the unlatched condition causes movement of the syringe clamp to the unclamped condition by releasing the closing force.

Aspect 17. The syringe pump assembly of Aspect 16, wherein the holding section includes a clamping surface and the syringe support includes a clamp bearing surface, wherein the clamping surface is configured to engage the clamp bearing surface such that movement of the holding section to the latched condition increases the closing force applied to the sealing section.

Aspect 18. The syringe pump assembly of Aspect 17, wherein the holding section further includes a clamping arm and the clamping surface is formed on the clamping arm and is configured to engage the clamp bearing surface at an underside of the syringe support in the latched condition.

Aspect 19. The syringe pump assembly according to any of Aspects 16-18, wherein the sealing element is a sealing o-ring.

Aspect 20. The syringe pump assembly according to Aspect 19, wherein the sealing section includes a pneumatic fitting, a channel fluidically connected to the pneumatic fitting and a channel opening arranged on the sealing surface and surrounded by the sealing o-ring.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A fluid processing system comprising:
at least one syringe having a barrel and a plunger configured for reciprocal movement within the barrel, wherein the syringe includes a top face and a top flange at a proximal end and a tip at a distal end;
a pump housing;
at least one syringe support arranged on the pump housing having a support surface configured engage the top flange to support the syringe in a fluid processing procedure; and
at least one syringe clamp connected to the housing, the syringe clamp comprising a sealing section connected to the housing with a first hinge and a holding section connected to the sealing section with a second hinge, the syringe clamp moveable between an unclamped condition and a clamped condition,
wherein the sealing section includes a sealing element on a sealing surface and is pivotable on the first hinge between a raised condition and a lowered condition in which the sealing section is configured to apply a clamping force to the top face of the syringe to form a seal against the top face with the sealing element,
wherein the holding section is pivotable on the second hinge between an unlatched condition and a latched condition engaged with the syringe support, and
wherein movement of the holding section to the latched condition causes movement of the syringe clamp to the clamped condition by applying a closing force to the sealing section through the second hinge such that the sealing section applies the clamping force to the top face, and movement of the holding section to the unlatched position causes movement of the syringe clamp to the unclamped condition by releasing the closing force and the clamping force.

2. The fluid processing system according to claim 1, wherein the holding section includes a clamping surface and the syringe support includes a clamp bearing surface,
wherein the clamping surface is configured to engage the clamp bearing surface such that movement of the holding section to the latched condition increases the clamping force applied by the sealing section.

3. The fluid processing system according to claim 2, wherein the holding section further includes a clamping arm and the clamping surface is formed on the clamping arm and is configured to engage the clamp bearing surface at an underside of the syringe support in the latched condition.

4. The fluid processing system according to claim 2, wherein:
the syringe support further includes spaced apart support flanges configured to receive the barrel therebetween, wherein the clamp bearing surface includes clamp bearing surfaces formed on the respective support flanges, and
the holding section further includes spaced apart clamping arms and the clamping surface includes clamping surfaces formed on the respective clamping arms, and
the clamping surfaces are configured to engage corresponding clamp bearing surfaces at an underside of the support flanges in the latched condition.

5. The fluid processing system according to any of claims 1-4, wherein the clamping surface includes a detent and the clamp bearing surface includes a bearing element, wherein the bearing element is positioned in the detent when the holding section is in the latched condition.

6. The fluid processing system according to any of claims 1-5, wherein the sealing element is a sealing o-ring, and
wherein the sealing o-ring is configured to be pressed against the top face under the clamping force in the clamped condition to form the seal.

7. The fluid processing system according to any of claims 1-5, wherein the sealing section includes a pneumatic fitting, a first channel fluidically connected to the pneumatic fitting and a first channel opening arranged on the sealing surface and surrounded by the sealing element preferably, wherein the top face includes a second channel having a second channel opening, and wherein the first channel and the second channel are fluidically connected in the clamped condition, more preferably, wherein air is provided to the syringe via the first channel and the second channel to pressurize the syringe between about -10 PSI and about 100 PSI.

8. The fluid processing system according to claim 7, comprising a plurality of syringes arranged on the pump housing.

9. The fluid processing system according to any of claims 1-7, wherein the tip includes a tip opening for fluid flow into or out of the barrel in response to pressurization of the syringe.

10. The fluid processing system according to any of claims 1-7, wherein the syringe is a disposable syringe.

11. The fluid processing system according to any of claims 1-7, further comprising a microfluidic chip fluidically connected to the syringe.

12. The fluid processing system according to claim 11, wherein the syringe is configured to be positively pressurized to drive fluid through the microfluidic chip.

13. The fluid processing system according to claim 11, wherein the syringe is configured to be negatively pressurized for priming the microfluidic chip.

14. A syringe pump assembly comprising:
a pump housing;
at least one syringe support arranged on the pump housing having a support surface configured to support a syringe in a fluid processing procedure; and
a corresponding at least one syringe clamp connected to the housing, the syringe clamp comprising a sealing section connected to the housing with a first hinge and a holding section connected to the sealing section with a second hinge, the syringe clamp moveable between an unclamped condition and a clamped condition,
wherein the sealing section includes a sealing element on a sealing surface and is pivotable on the first hinge between a raised condition corresponding to the unclamped condition and a lowered condition,
wherein the holding section is pivotable on the second hinge between an unlatched condition corresponding to the unclamped condition and a latched condition engaged with the syringe support and corresponding to the clamped condition, and
wherein movement of the holding section to the latched condition causes movement of the syringe clamp to the clamped condition by applying a closing force to the sealing section through the second hinge, and movement of the holding section to the unlatched condition causes movement of the syringe clamp to the unclamped condition by releasing the closing force.

15. The syringe pump assembly of claim 14, wherein the holding section includes a clamping surface and the syringe support includes a clamp bearing surface,
wherein the clamping surface is configured to engage the clamp bearing surface such that movement of the holding section to the latched condition increases the closing force applied to the sealing section, preferably, wherein the holding section further includes a clamping arm and the clamping surface is formed on the clamping arm and is configured to engage the clamp bearing surface at an underside of the syringe support in the latched condition.

16. The syringe pump assembly according to any of claims 14-15, wherein the sealing element is a sealing o-ring, preferably, wherein the sealing section includes a pneumatic fitting, a channel fluidically connected to the pneumatic fitting and a channel opening arranged on the sealing surface and surrounded by the sealing o-ring.
